(19) **Europäisches Patentamt / European Patent Office / Office européen des brevets**

(11) **EP 3 999 665 B1**

(12) ## EUROPEAN PATENT SPECIFICATION

(45) Date of publication and mention of the grant of the patent:
**10.05.2023 Bulletin 2023/19**

(21) Application number: **20735214.7**

(22) Date of filing: **06.07.2020**

(51) International Patent Classification (IPC):
**C12Q 1/689** (2018.01)

(52) Cooperative Patent Classification (CPC):
**C12Q 1/689; C12Q 2600/158**

(86) International application number:
**PCT/EP2020/068980**

(87) International publication number:
**WO 2021/008920 (21.01.2021 Gazette 2021/03)**

(54) **METHOD FOR DETERMINING THE RISK OF AN AVIAN PATHOGENIC E. COLI (APEC) INFECTION IN AN AVIAN FLOCK**

VERFAHREN ZUR BESTIMMUNG DES RISIKOS EINER GEFLÜGELPATHOGENEN E. COLI(APEC)-INFEKTION IN EINER GEFLÜGELSCHAR

PROCÉDÉ PERMETTANT DE DÉTERMINER LE RISQUE D'INFECTION PAR LA SOUCHE E. COLI PATHOGÈNE AVIAIRE (APEC) DANS UN TROUPEAU AVIAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2019 EP 19186697**

(43) Date of publication of application:
**25.05.2022 Bulletin 2022/21**

(73) Proprietor: **Evonik Operations GmbH**
**45128 Essen (DE)**

(72) Inventors:
- **DARGATZ, Michelle**
  **33604 Bielefeld (DE)**
- **THIEMANN, Frank**
  **48031 Nottuln (DE)**
- **WEISSMANN, Michaela**
  **32130 Enger (DE)**
- **PELZER, Stefan**
  **33335 Gütersloh (DE)**
- **FLÜGEL, Monika**
  **33803 Steinhagen (DE)**
- **BÖHL, Florian**
  **60151 Neckargemünd (DE)**
- **KAPPEL, Andreas**
  **61479 Glashütten (DE)**
- **IGWE, Emeka Ignatius**
  **80333 München (DE)**

- **RETH, Alexander**
  **33615 Bielefeld (DE)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
**WO-A1-2016/179027      US-A1- 2004 219 530**

- **T. J. JOHNSON ET AL: "Identification of Minimal Predictors of Avian Pathogenic Escherichia coli Virulence for Use as a Rapid Diagnostic Tool", JOURNAL OF CLINICAL MICROBIOLOGY, vol. 46, no. 12, 8 October 2008 (2008-10-08), pages 3987-3996, XP055659702, US ISSN: 0095-1137, DOI: 10.1128/JCM.00816-08**
- **WALKER DAVID I ET AL: "A highly specificEscherichia coliqPCR and its comparison with existing methods for environmental waters", WATER RESEARCH, vol. 126, 25 August 2017 (2017-08-25), pages 101-110, XP085246911, ISSN: 0043-1354, DOI: 10.1016/J.WATRES.2017.08.032**

- **CSABA VARGA ET AL: "Evaluating Virulence-Associated Genes and Antimicrobial Resistance of Avian Pathogenic Escherichia coli Isolates from Broiler and Broiler Breeder Chickens in Ontario, Canada", AVIAN DISEASES., vol. 62, no. 3, 4 May 2018 (2018-05-04), pages 291-299, XP055653750, US ISSN: 0005-2086, DOI: 10.1637/11834-032818-Reg.1**

Remarks:

The complete document including Reference Table(s) and the Sequence Listing(s) can be downloaded from the EPO website

**Description**

Field of the Invention

The present invention pertains to a method for determining the risk of an APEC infection in an avian flock. The method is based on determining the quantitative ratio of the amount of at least virulence factors iroN and ompT, each with respect to the amount of an E.coli reference gene in a pooled sample deriving from said avian flock.

Background of the Invention

Colibacillosis causes the largest disease and mortality losses in poultry production worldwide. The high diversity of the responsible pathogen Escherichia coli complicates both the diagnosis of APEC and the development of a vaccine against the pathogen. E. coli is an integral part of a healthy intestinal microbiome and only strains with certain properties, so-called APECs (avian pathogenic E. coli), trigger various clinical pictures such as respiratory problems, fallopian tube infections, yolk sac inflammations or Swollen Head Syndromes. Pathogenic strains have virulence factors that allow them to invade the host and degrade the tissue. These virulence factors (VFs) are potential biomarkers for the diagnosis of APEC, but they vary across different APEC isolates and also non-pathogenic strains may carry individual VFs. Therefore, APEC cannot be diagnosed by single VFs and must be detected by a panel of markers.

Current diagnostic methods are based on the selective isolation of E. coli from colibacillosis-typical lesions in visceral tissues such as liver, spleen and lung after a section. The isolates are then classified as pathogenic or non-pathogenic by the PCR-based detection of various VFs.

These methods however are not only time and cost consuming but also do not allow a conclusion on the whole flock APEC status. Known PCR assays allowing the discrimination between APEC and non-APEC usually rely on the detection of a set of virulence factors with a defined threshold for a minimum number of VFs. An example for such method is a five-VF PCR published by Johnson (Johnson, T. J., et al. (2008). "Identification of Minimal Predictors of Avian Pathogenic Escherichia coli Virulence for Use as a Rapid Diagnostic Tool." J Clin Microbiol 46(12): 3987-3996.), where a strain is defined as an APEC as soon as it carries minimum four out of five factors.

However, a rapid, non-invasive method for the detection of APEC or for determining the APEC risk is not known. For whole flock evaluation, a method for detecting APEC in DNA obtained from pooled fecal flock samples would be particularly desirable. The development of such method is particularly complex as (i) coccidiosis results in diverse clinical symptoms ranging from salphingitis to Swollen Head Syndrome (meningitis) but not typically in a gut inflammatory problem, which makes the detection of the pathogen in feces unapparent; and (ii) APEC virulence factors are generally not suited for APEC detection in non-pure culture samples, as the identical markers occur in several close related organisms other than *E. coli* due the partly poor genetically segregation of the species (Schouler, C., et al. (2012). "Diagnostic strategy for identifying avian pathogenic Escherichia coli based on four patterns of virulence genes." J Clin Microbiol 50(5): 1673-1678; Johnson, T. J., et al. (2008). "Identification of Minimal Predictors of Avian Pathogenic Escherichia coli Virulence for Use as a Rapid Diagnostic Tool." J Clin Microbiol 46(12): 3987-3996.). Also, selected virulence factors might occur in different strains contained in the fecal sample, giving the result of a critical number of factors when taken together. Thus, a simple PCR detection of APEC virulence factors in feces would not allow a conclusion on the APEC risk or on APEC infections.

In view of the above, it was the objective of the present invention to provide a fast and reliable, non-invasive, ante mortem in-process method for determining the risk of an APEC infection in an avian flock, which can be performed at low cost and with minimal effort.

Summary of the Invention

This objective is solved by a method for determining the risk of an Avian Pathogenic E. coli (APEC) infection in an avian flock, the method comprising determining the quantitative ratios of the amount of specific virulence factors (VF), each with respect to the amount of E. coli reference gene is ybbW (SEQ ID NO: 6) or functional fragments thereof, in a pooled sample deriving from the avian flock, wherein the specific virulence factors include at least the genes iroN (SEQ ID NO.: 1) and ompT (SEQ ID NO: 2) or functional fragments thereof.

Detailed Description of the Invention

The inventors have unexpectedly found that the risk of an APEC infection in an avian flock can be determined on the basis of the quantitative ratios of the amounts of the specific virulence factors iroN (SEQ ID NO.: 1) and ompT (SEQ ID NO: 2), each with respect to the amount of E. coli reference gene is ybbW (SEQ ID NO: 6) in a pooled sample deriving from said avian flock.

That is, the combined quantitative ratios of the amounts of marker genes iroN/ ybbW and ompT/ ybbW contained in a pooled sample from an avian flock allow an APEC-risk evaluation for said avian flock. The APEC-risk evaluation is based on the finding that during an APEC infection a clonal pathogenic strain carrying virulence factors encoded on the colV plasmid becomes dominant and outpaces non-pathogenic commensal E. colis. In such a case, the ratio of VFs/ybbW will increase significantly during the disease progression and VFs counts in non-pathogenic or non E. coli strains become neglectable.

The virulence factors, and functional fragments thereof, respectively, are detected on DNA level. The same applies for the E.coli reference gene.

The specific virulence factors may further include at least one of hlyF (SEQ ID NO: 3), iss (SEQ ID NO: 4), iutA (SEQ ID NO: 5), or functional fragments thereof. Accordingly, in these embodiments according to the present invention, the method involves determining the quantitative ratios of the amounts of iroN/ ybbW and ompT/ ybbW, plus at least one of the ratios of the amounts of hlyF/ ybbW, iss/ ybbW, and iutA/ ybbW.

The APEC virulence factors and E. coli reference gene according to the present invention are summarized below:

| Gene Symbol | Synonyms | NCBI GeneID | Protein | NCBI Reference Sequence | Reference Strain | Location | Target Organism (s) |
|---|---|---|---|---|---|---|---|
| *iroN* SEQ ID NO: 1 | fepA_1, fepA_2, fepA_3, pfeA, *iroN2* | 3853509 | *iroN*-related siderophore receptor | NC_007675.1 | *Escherichia coli* (strain: A2363) | 148717-150894 | *Escherichia coli* |
| *ompT* SEQ ID NO: 2 | *ompT*1 | 3853531 | outer membrane protein; protease precursor | NC_007675.1 | *Escherichia* coli (strain: A2363) | 114321-115274 | *Escherichia coli* |
| *hlyF* SEQ ID NO: 3 | - | 9846088 | hemolysin *HlyF* | NC_014615.1 | *Escherichia coli* (strain: 0102-ST405) | 115235-116344 | *Escherichia coli* |
| *iss* SEQ ID NO: 4 | - | 3853500 | involved in increased serum survival and complement resistance | NC_007675.1 | *Escherichia coli* (strain: A2363) | 137931-138239 | *Escherichia coli* |
| *iutA* SEQ ID NO: 5 | - | 3853520 | ferric aerobactin receptor | NC_007675.1 | *Escherichia coli* (strain: A2363) | 103916-106117 | *Escherichia coli* |
| *ybbW* SEQ ID NO: 6 | glxB2 | 945138 | putative allantoin transporter | NC_000913.3 | *Escherichia coli* str. K-12 substr. MG1655 (strain: K-12, substrain: MG1655) | 537633-539087 | *Escherichia coli* |

The avian flock according to the invention is preferably poultry. Preferred poultry according to the invention are chickens, turkeys, ducks and geese. The poultry can be optimized for producing young stock. This type of poultry is also referred to as parent and grandparent animals. Preferred parent and grandparent animals are, accordingly, (grand)parent broilers, (grand)parent ducks, (grand)parent turkeys and (grand)parent geese.

The poultry according to the invention can also be selected from fancy poultry and wild fowl. Preferred fancy poultry or wild fowl are peacocks, pheasants, partridges, guinea fowl, quails, capercailzies, goose, pigeons and swans. Further preferred poultry according to the invention are ostriches and parrots. Most preferred poultry according to the invention are broilers.

The pooled sample deriving from the avian flock preferably is an environmental sample collected in the stable housing the avian flock. The term "environmental sample" is to be understood as a sample that is sufficient to characterize the immediate environment of the avian flock and is retrieved from the stable housing of the flock in a non-invasive manner.

Examples for such environmental samples are excremental material, litter material, air dust material or combinations thereof. In general, the term "litter" is to be understood as a mixture of avian excrements with the bedding material.

In the context of the present invention, the term "air dust" is to be understood as airborne particles that are found in the immediate environment of the avian flock within the stable housing.

Air dust samples are retrieved by application of airborne particle collecting devices (electrified or non-electrified) and are processed by the following general steps: washout of the sample, optional lysis of the cells and purification of DNA, application of the washout, lysate or purified DNA as a template for PCR. The sample washout is performed by flooding and incubating the collection vessel, filter, swap or cartridge with a sterile aqueous solution at neutral pH such as water or 10 mM TE buffer or with a lysis buffer containing chaotropic salts to chemically disrupt cells and to stabilize and protect nucleic acids against nucleases in solution. To improve the recovery of dust particles, shaking is applied during the washout. The solved sample is retrieved from the collection device and depending on the sample quality and pureness, it is either is directly applied as a template for PCR or further processed. If the sample is further processed, it is lysed by heating and disrupted by application of bead beating. For DNA purification, the lysed sample can be applied preferably to a standard silica based DNA extraction kit or other methods for DNA extraction such as reverse chromatography, Phenol Chloroform Isoamylalcohol extraction or salting out. The DNA eluate is used as a template for PCR.

Preferably, the environmental sample is excremental material, in particular fecal material.

The fecal material may be a composite sample of randomly collected individual fecal samples.

The excremental samples to be taken from a specific avian flock are ideally taken at a discrete number of sites within the animal house in order to obtain a pooled sample being representative for the avian population as a whole.

The sample size (i.e. the number of excremental samples to be taken; each sample taken at a specific site within the animal house) has to be determined in view of the actual stocking density, i.e. with the actual number of animals belonging to the avian population to be tested.

The sample size may be calculated using the following formula:

$$n_0 = \frac{Z^2 pq}{e^2}$$

wherein

$n_0$ is the sample size recommendation

Z is 1.96 for 95% confidence level

p is the estimated portion of the population with the attribute in question q is 1-p, and e is the confidence interval expressed as decimal.

In general, a minimum of 80 to 100 individual excremental samples are sufficient for most livestock avian populations. Broilers are usually kept in flocks which can consist of > 20000 birds in one house.

As an example, for a broiler flock of 20000 animals, 96 individual samples are required for a confidence level of 95%.

The above formula is particularly suitable for determining the sample size required for large population.

For smaller populations, (<= 100), the sample size recommendation $n_0$ as obtained with the above formula may be further adjusted in accordance with the following formula:

$$n = \frac{n_0}{1 + \frac{(n_0 - 1)}{N}}$$

wherein

N is the population size, and
n is the adjusted sample size.

For obtaining the pooled excremental sample material as required in step (a), several sampling methods may be used.

In one embodiment, the pooled excremental sample may be obtained by systematic grid sampling (systematic random sampling). For this method, the area in which the avian population is kept is divided in a grid pattern of uniform cells or sub-areas based on the desired number of individual excremental samples (i.e. the sample size). Then, a random sample collection site is identified within the first grid cell and a first sample is taken at said site. Finally, further samples are obtained from adjacent cells sequentially - e.g. in a serpentine, angular or zig-zag fashion - using the same relative location within each cell. A random starting point can be obtained with a dice or a random number generator.

The above process may optionally be repeated for replicate samples. That is, a new random position is established for the single collection point to be repeated in all of the cells. By analyzing replicate samples, variabilities in the estimate of the mean provided by the original samples may be determined.

The sample size corresponds to the number of cells in the grid pattern in case one sample is to be taken per cell. In general, in case x samples are to be taken per cell, the sample size is the number of cells, divided by x.

The systematic grid sampling method can be easily implemented in the field. Thereby, over- or underrepresentation of subareas can be avoided

Another sampling method is stratified random sampling (i.e. random sampling within a grid). Herein, samples are obtained sequentially from adjacent grid cells, but the location of the sample within each cell is random.

Alternatively, the samples may be taken by simple random sampling, where the samples are taken from random locations (without gridding) across the area in which the animals are kept. For this method, a formal approach for determining the random sample locations must be used, e.g. based upon a random number generator.

The samples may be collected manually with a spatula or a similar device and are immediately transferred into a sample collection vessel or tube.

In an alternative embodiment, the pooled excremental sample may be obtained using the overshoe method while walking through the house using a route that will produce representative samples for all parts of the house or the respective sector. Such route may e.g. be uniformly shaped serpentines or sinuous lines, angular lines or zigzag lines. Boot swabs being sufficiently absorptive to soak up moisture are particularly suitable. However, tube gauze socks are also acceptable.

Suitable sample volumes are, for example, 0.1 to 20 ml, in particular 0.2 to 10 ml, preferably 0.5 to 5 ml. Suitable sample masses are, for example, 0.1 to 20 g, in particular 0.2 to 10 g, preferably 0.5 to 5 g.

The thus-obtained sample material may comprise heterogeneous components such as used feed or litter material and thus has to be homogenized. The skilled person is aware of suitable, commonly used homogenization techniques.

The homogenized sample material is preferably diluted and stabilized with aqueous buffer. This buffer preferably comprises buffer solution, detergents, denaturing agent and/or complexing agents. Advantageously, said aqueous buffer solution comprises chaotropic salts to chemically disrupt cells and to stabilize and protect nucleic acids against nucleases in solution. Optionally, the aqueous buffer solution further comprises nuclease inhibitors.

The cell material contained in the diluted sample material is lysed. Lysis may be performed via chemical lysis or via mechanical lysis. Chemical lysis reagents are, for example, guanidiniumthiocyanat. Mechanical lysis may be accomplished by treating the sample with beads, ultrasonic or ultra turrax®. Advantageously, the lysis step comprises both, a mechanical lysis treatment and a chemical lysis treatment.

Thereafter, nucleic acid material is isolated, for example by magnetic bead extraction. The skilled artisan is aware of additional or alternative nucleic acid isolation techniques.

The detection and quantification of the virulence factors may be performed for example via PCR, qPCR, sequencing or hybridization techniques. preferably via qPCR.

For quantification, external calibrated quantification standards are used. Results are indicated as copies/$\mu$l (copies/g feces).

The qPCR-based detection method according to the present invention may include multiplex amplification of a plurality of markers or target genes simultaneously. It is well known in the art to select PCR primers to generate PCR products that do not overlap in size and can be analyzed simultaneously.

The present invention further provides an environmental screening kit comprising a set of oligonucleotides (primers and probes) for amplifying and quantifying at least virulence factors iroN, ompT and E.coli reference gene ybbW. In one embodiment, the environmental screening kit comprises primers and optionally comprising probes for detecting and quantifying at least iroN (SEQ ID NO.: 1), ompT (SEQ ID NO: 2), and ybbW(SEQ ID NO: 6). Said environmental screening kit may further comprise primers and optionally probes for detecting and quantifying at least one gene selected from the group consisting of hlyF (SEQ ID NO: 3), iss (SEQ ID NO: 4), iutA (SEQ ID NO: 5). The kit may further comprise buffer solutions, such as PCR buffer; magnesia salts; deoxy nucleotide triphosphates (dNTPs). The kit may also include elements such as sample collection tubes, reagents to isolate the nucleic acids (DNA) or instructions for its use.

Applications of the method according to the invention are for example (i) aiding in determining the risk of APEC infections or diagnosis of APEC infections, (ii) monitoring the progress or reoccurrence of APEC infections, and (iii) aiding in the evaluation of treatment efficacy for an avian flock undergoing or contemplating treatment.

Applications of the invention particularly help to avoid loss in animal performance like weight gain and feed conversion.

Brief description of the Figures

**Fig. 1:** Agarose gel containing pentaplex PCR products from fecal samples collected from an *E. coli* outbreak flock (lane 1.1-4.1), a healthy flock (lane -K1.1- -K2.2) and the negative Non-Template control (NTC)

**Fig. 2:** Agarose gel containing pentaplex PCR products from 16 different *E. coli* clones isolated from feces from an *E. coli* outbreak (Lane 1-16), positive control (+K, positive fecal DNA) and NTC (H2O)

**Fig. 3:** Results for the iss and *iroN* qPCR in logarithmic Starting Quantity (SQ) and for *ybbW* in Quantification Cycles (Cq) for fecal samples from an *E. coli* outbreak and a healthy Flock

**Fig. 4:** Agarose gel containing pentaplex PCR products from different fecal field samples, the positive control and water control (NTC)

**Fig. 5:** Results for the *ybbW* qPCR in Quantification Cycles (Cq) for 11 field samples and the positive control

**Fig. 6:** Agarose gel containing pentaplex PCR products from dust samples collected from an *E. coli* outbreak flock (lane 1-4), two positive fecal samples (lane 5,6) and the negative Non-Template control (NTC, lane 7)

In the following, the invention is illustrated by non-limiting examples and exemplifying embodiments.

Examples

**MATERIAL AND METHODS**

ISOLATION OF *E. COLI* FROM FECES

For isolation of *E. coli* from fecal sample, one shade point of feces was filled into 10 ml Casein-Peptone Soymeal-Peptone (CASO) Bouillon and incubated for 10 min at 37° C. Afterwards, 100 $\mu$l were plated onto Eosin Methylene Blue (EMB) Agar, a selective medium for *E. coli.* The plates were incubated at 37° C over night and examined for colonies with a green metallic sheen, which is typical for *E. coli.* Isolated strains were further characterized by screening for virulence factors (see below).

DNA ISOLATION

FROM FECAL SAMPLES

Fecal samples were filled into ScreenFloX® PCR Sample collection tubes and pretreated according to the ScreenFlox Sample collection tubes and Nucleic acid extraction Instructions for Use (IFU). The extraction of nucleic acids from the pretreated samples was conducted with the ScreenFloX® PCR Nucleic acid extraction kit according to the IFU.

FROM BACTERIAL CULTURES

Nucleic acid extraction from bacteria was performed using overnight cultures of the organisms in their standard medium. 600 µl of the overnight culture was incubated together with 600 µl lysis buffer from the ScreenFlox® PCR Nucleic acid extraction kit at 70° C for 20 min. 500 µl of the lysed culture were applied for DNA extraction with the ScreenFlox® PCR Nucleic acid extraction kit according to the IFU.

PENTAPLEX PCR

Samples were tested for the presence of five APEC pathogenicity markers according to the method from Johnson (2008) by applying the following primers in a combined PCR reaction:

TABLE 1 PRIMERS AND AMPLICON SIZES FOR THE PENTAPLEX **PCR** (JOHNSON **2008**)

| Target | Primer forward (5'-3') | Primer reverse (5'-3') | Amplicon Size |
|---|---|---|---|
| *iroN* | AATCCGGCAAAGAGACGAACCGCCT SEQ ID NO: 7 | GTTCGGGCAACCCCTGCTTTGACTTT SEQ ID NO: 8 | 553 |
| *ompT* | TCATCCCGGAAGCCTCCCTCACTACTAT SEQ ID NO: 9 | TAGCGTTTGCTGCACTGGCTTCTGATAC SEQ ID NO: 10 | 496 |
| *hlyF* | GGCCACAGTCGTTTAGGGTGCTTACC SEQ ID NO: 11 | GGCGGTTTAGGCATTCCGATACTCAG SEQ ID NO: 12 | 450 |
| *iss* | CAGCAACCCGAACCACTTGATG SEQ ID NO: 13 | AGCATTGCCAGAGCGGCAGAA SEQ ID NO: 14 | 323 |
| *iutA* | GGCTGGACATCATGGGAACTGG SEQ ID NO: 15 | CGTCGGGAACGGGTAGAATCG SEQ ID NO: 16 | 302 |

The PCR was conducted using the Phusion High-Fidelity DNA Polymerase (Thermo Scientific). Each reaction was set up according to the following table:

**TABLE 2 REACTION SET UP FOR THE PENTAPLEX PCR**

| Reagent | Volume per reaction [µl] |
|---|---|
| Buffer (5x) | 4 |
| d NTPs (10 mM stock) | 0.2 |
| Each primer fw (10 µm stock) | 0.3 (1.5 total) |
| Each Primer rv (10 µM stock) | 0.3 (1.5 total) |
| DNA template | 2 |
| DMSO | 0.6 |
| Phusion DNA polymerase | 0.2 |
| PCR-grade water | 9.8 |
| **Total volume** | **20µl** |

Amplification in a thermal cycler was conducted according to Table 3:

**TABLE 3 THERMAL CYCLING PROGRAM FOR THE PENTAPLEX PCR**

| Reaction | Time (min) | Temp (°C) | Repeats |
|---|---|---|---|
| Initial denaturing | 2:00 | 98 | 1x |
| Denaturing | 0:30 | 98 | 30x |
| Annealing/ | 0:30 | 63 | |
| Elongation | 0:30 | 68 | |
| Final elongation | 10:00 | 72 | 1x |
| Hold | - | 4 | |

Amplificated samples were analyzed in a 2% agarose gel. Referring to Johnson (2008), an *E. coli* strain was classified as an APEC strain as soon as it carried at least 4 out of 5 virulence factors.

*ISS* AND *IRON* QPCR

For the two APEC targets iss and *iroN,* quantitative real-time PCRs (qPCRs) were developed. Both assays showed a PCR-efficiency of 100-105% and a linear range from 10 to $10^8$ copies/µl. Following primers and probes were designed to detect both targets:

**TABLE 4 PRIMER AND PROBES FOR THE ISS AND IRON QPCR**

| Target | Primer forward (5'-3') | Primer reverse (5'-3') | Probe (5'-3') | Fluoropho re | Quencher |
|---|---|---|---|---|---|
| *iroN* | GCGCACTGATTT GAGAATGA    SEQ ID NO: 17 | CTTCCTCTACCA GCCTGACG    SEQ ID NO: 18 | CGATCCGCA GCAGAGCCA GA    SEQ ID NO: 19 | Cy5 | BHQ2 |
| *iss* | CGGGAATTGGAC AAGAGAAA    SEQ ID NO: 20 | ATATACCCGGGC TTCCAAAC    SEQ ID NO: 21 | ATGCAGCCA AAATTTGTG GCGG    SEQ ID NO: 22 | FAM | BHQ1 |

qPCR Reactions were set up with the iTaq™ Universal Probes Supermix (Bio-Rad) according to Table 5:

TABLE 5 REACTION SET-UP FOR THE Iss AND *IRON* QPCR

| Component | Volume per reaction [μl] |
|---|---|
| iTaq™ Universal Probes Supermix | 10 |
| Primer for (20 μM stock) | 0.5 |
| Primer rev (20 μM stock) | 0.5 |
| Probe (10 μM stock) | 0.5 |
| Template | 1 |
| PCR-grade water | 7.5 |
| **Total volume** | 20 |

The qPCRs were performed in a Bio-Rad CFX96 cycler using the following cycling protocol under detection of all channels:

TABLE 6 CYCLING PROTOCOL FOR THE *ISS* AND *IRON* QPCR

| Step | Temp | Time | Repeats |
|---|---|---|---|
| **Initial Denaturation** | 95° C | 3 min | 1x |
| **Denaturation** | 95° C | 5 s | 40x |
| **Annealing/Extension + read** | 60° C | 30 s | |

Each run contained quantification standard with known concentrations of the target ranging from 10 to $10^4$ copies/μl. The standard curve was used to calculate the concentration of iss and *iroN* from the quantification cycle (Cq) in each sample.

*YBBW* QPCR

The qPCR for the E. *coli ybbW* reference gene was conducted according to the method published by Walker et al (Walker, D. I., et al. (2017). "A highly specific Escherichia coli qPCR and its comparison with existing methods for environmental waters." Water Research 126: 101-110). Following published primers were used:

TABLE 7 PRIMERS FOR THE *YBBW* QPCR (WALKER 2017)

| Target | Primer forward | Primer reverse |
|---|---|---|
| **ybbW** | TGATTGGCAAAATCTGGCCG          SEQ ID NO: 23 | GAAATCGCCCAAATCGCCAT          SEQ ID NO: 24 |

qPCR Reactions were set up with iTaq™ Universal SYBR® Green Supermix (Bio-Rad) according to the following table:

TABLE 8 REACTION SET-UP FOR THE ISS AND *IRON* QPCR

| Component | Volume per reaction [μl] |
|---|---|
| iTaq™ Universal SYBR® Green Supermix | 10 |
| Primer for (20 μM stock) | 0.5 |
| Primer rev (20 μM stock) | 0.5 |
| Template | 1 |
| PCR-grade water | 8 |

(continued)

| Component | Volume per reaction [μl] |
|---|---|
| Total volume | 20 |

The qPCR was performed in the Bio-Rad CFX96 cycler using the following cycling protocol under detection the SYBR channel:

**TABLE 9 CYCLING PROTOCOL FOR THE *YBBW* QPCR**

| Step | Temp | Time | Repeats |
|---|---|---|---|
| Initial Denaturation | 95° C | 2 min | 1x |
| Denaturation | 95° C | 5s | 40x |
| Annealing/Extension + read | 60° C | 30 s | |
| Melt curve | 65°-95° C | Increment 0.5° C/ 5 s | 1x |

For this assay no quantification standards were used, thus the results were interpreted by the quantification cycle (Cq) only. The higher a Cq, the lower is the starting quantity (SQ) of the target in the reaction, whereby a difference of three Cq equals an approximate SQ difference of one log.

## RESULTS AND DISCUSSION

FECAL SAMPLES FROM APEC BREAKOUT

PENTAPLEX PCR

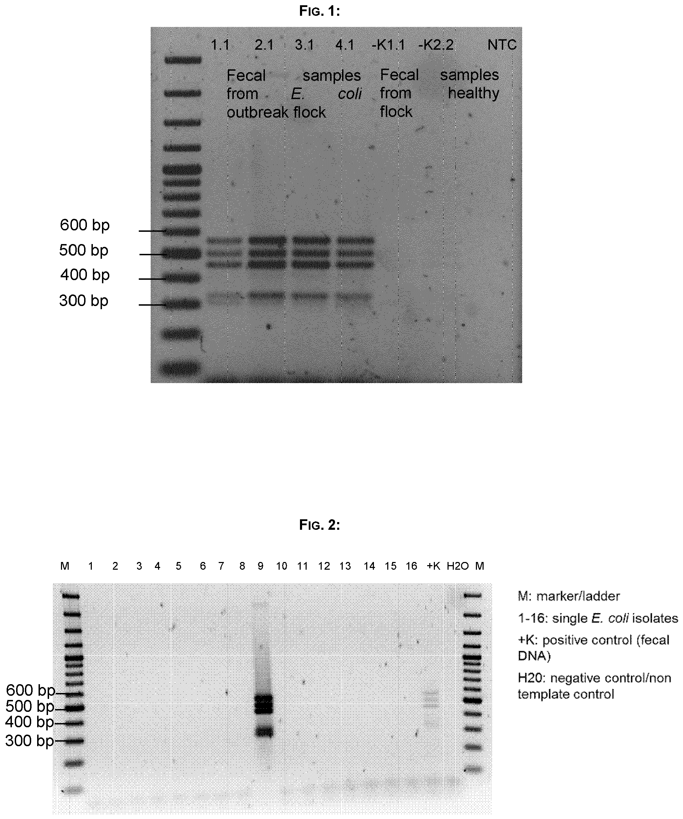

Fecal samples were collected from a commercial broiler flock in Germany diagnosed with an APEC outbreak diagnosed with polyarthritis and compared to samples collected from a commercial flock from the same farm without any clinical signs. The samples were evaluated with the APEC Pentaplex PCR (Johnson, 2008). The resulting agarose gel is depicted in Figure 1. All five APEC targets were clearly detected in the four fecal samples received from the outbreak flock (lane 1.1-4.1). For the samples from the healthy flock, no or only very faint bands are visible. The negative control did not show any PCR amplifications.

From these results it is clearly visible that the published APEC virulence factors cannot only be used for characterization of *E. coli* isolates, but also for detecting virulence factors in DNA isolated from fecal samples of infected birds, even though no intestinal problems but polyarthritis was diagnosed. Also, a clear differentiation of fecal samples from an infected and from a healthy flock is possible for this sample set.

VIRULENCE FACTOR TYPING OF *E. COLI* CLONES

The fecal sample collected from the outbreak flock, containing all 5 VFs was further analyzed. By VF typing of single colonies isolated from this sample it was evaluated if

- the virulence factors, found in the total fecal DNA, originated from different strains carrying subsets of the factors

- or from single pathogenic strains carrying all five factors.

*E. coli* was isolated by selective cultivation and a subset of strains was applied virulence factor typing according to the classification scheme from Johnson (2008).

Overall, 16 fecal isolates were screened and only one out of 16 isolates (6.25%) showed positive results (Figure 2, lane 9). All 5 markers were detected highly positive in that one isolate. According to the Johnson (2008) APEC characterization, only this clone is defined as an avian pathogenic strain. All other isolates are non-pathogenic.

From the virulence factor typing of the *E. coli* isolates it becomes clear that the five virulence factors detected in the whole fecal DNA probably originate from single pathogenic strains, carrying all virulence genes and inducing the infection in the birds. No strains carrying only three or less factors, resulting in a total count of five factors when extracted together, were found. This finding is important, as false positive detection due to several strains carrying less than four genes but resulting in four or more positive APEC VFs when analyzed together, presents high risk for evaluating total fecal DNA.

*ISS*, *IRO*N AND *YBBW* QPCR

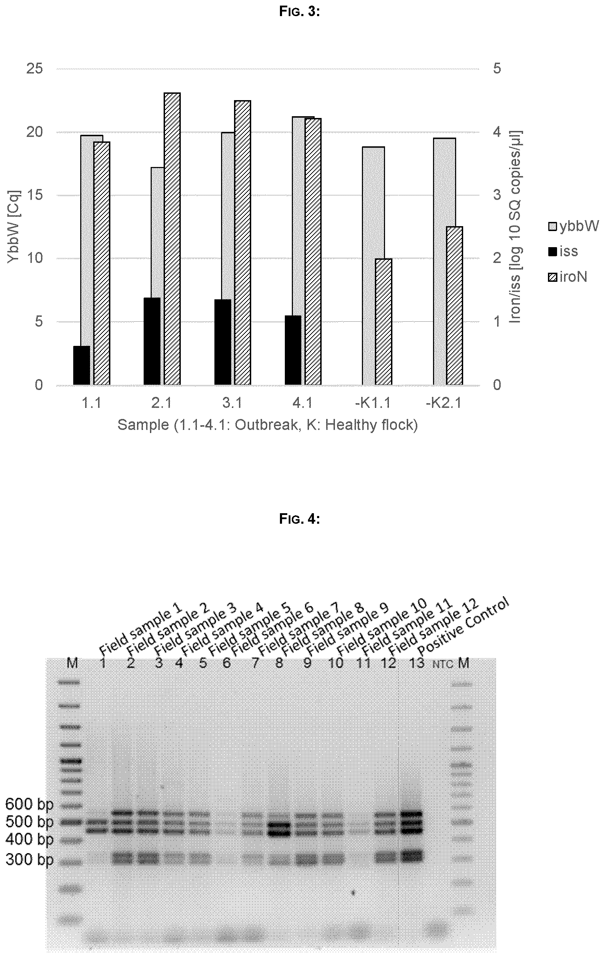

The same fecal samples collected from the E. *coli* outbreak and the healthy flock were analyzed regarding their load of iss, *iroN* and *ybbW* using the qPCR assays described above. The results for the logarithmic starting quantity of iss and *iroN* and the Cq of *ybbW* are shown in Figure 3.

While iss was detected in samples from the outbreak (1.1-4.1) only, *iroN* was found in all samples but with an approximately 100x lower load in the samples from the healthy flock (-K1.1- -K2.2). The *E. coli* reference gene *ybbW* was detected with no significant Cq variations over the two sample origins (standard deviation of 1.22 Cq), indicating a low variation of the abundance of this gene in the different samples.

The results from this qPCR analysis reveal important findings relevant for the proposed assay design. The *E. coli* reference marker *ybbW* was found with a similar abundance in fecal samples from healthy and from infected *E. coli* flocks, while the concentration of the APEC pathogenicity factors iss and *iroN* increase significantly in the outbreak sample. Consequently, the amount of pathogenic *E. coli* strains increased during the APEC pathogenesis, while the overall load of *E. coli* seems to be constant. A quantitative relation of pathogenic strains to overall *E. coli* in fecal samples identify flocks with a high risk of APEC infection or can confirm an APEC outbreak when performance losses occur.

ANALYSIS OF FIELD- AND TRIAL SAMPLES

SAMPLES FROM *SALMONELLA* AND *CAMPYLOBACTER* INFECTION TRIAL

Fecal samples from a *Salmonella* and *Campylobacter* infection trial were analyzed with the APEC pentaplex PCR. From each group *(Salmonella* infected, non-infected and *Campylobacter* infected), 12 biological replicates in forms of single bird samples from the same trial day were tested with the pentaplex PCR.

The results are depicted in Table 10.

Overall, the five virulence factors were very abundant over the tested samples (81% of the samples contained all five VFs). Interestingly, none of the samples contained only four or less VFs. Also, a higher portion of APEC positive birds was found in the *Salmonella* and *Campylobacter* infection groups (every sample with a VF count ≥4 was assumed to be APEC positive).

**TABLE 10 SUMMARIZED APEC VF COUNT IN 36 SINGLE BIRD FECAL SAMPLES RECEIVED FROM A *SALMONELLA* AND *CAMPYLOBACTER* INFECTION TRIAL**

| Treatment group | Virulence factor occurrence | | | | | | APEC occurrence (min 4/5) |
|---|---|---|---|---|---|---|---|
| | 0/5 | 1/5 | 2/5 | 3/5 | 4/5 | 5/5 | |
| *Salmonella infection* | 0% | 0% | 0% | 0% | 0% | 100% | 100% |
| *Non-infected* | 0% | 0% | 0% | 0% | 0% | 58% | 58% |
| *Campylobacter infection* | 0% | 0% | 0% | 0% | 0% | 83% | 83% |

These results indicate a relation between APEC occurrence and other diseases, such as Salmonellosis or Campylobacteriosis. In the literature, colibacillosis is often described as a secondary infection, occurring in immune suppressed birds suffering from a primary infection with another pathogen (Schouler, C., et al. (2012). "Diagnostic strategy for identifying avian pathogenic Escherichia coli based on four patterns of virulence genes." J Clin Microbiol 50(5): 1673-1678).

FIELD SAMPLES

Fecal field samples from commercial US broiler flocks with unknown health status were analyzed regarding the occurrence of APEC markers as well as their load with *ybbW.*

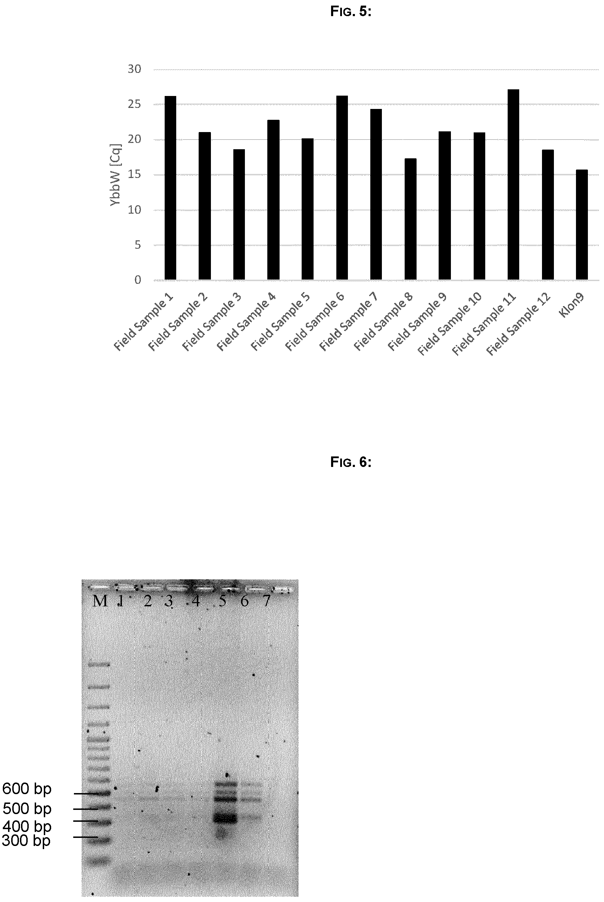

The resulting agarose gel containing the amplificates from the APEC pentaplex PCR is shown in Figure 4. All five virulence factors were found in all 11 samples, however different quantities were amplified indicated by the differences in band intensities. *YbbW* was also detected in all samples (Figure 5), with a rather low variation. The maximum difference of in Cq was approximately 3, which equals 1 log difference copy numbers.

The results from the analysis of the field and trial samples emphasize that all APEC VFs as well as the *ybbW* reference gene are very abundant in broiler fecal samples, however differences in the concentration, especially for the APEC VFs are found. Thus, the sole detection of a VF set without quantification does not allow a discrimination between infected and non-infected flocks, as the VFs occur with a high prevalence at least with a low concentration. However, a quantitative method that allows not only the numeric count of APEC factors, but also the relation of the factor concentration to the overall E. *coli* load can give insight into the APEC risk of the flock.

INCLUSIVITY AND EXCLUSIVITY TESTS OF THE *YBBW* QPCR

In order to evaluate the suitability of the *ybbW* gene as an E. *coli* reference gene, the cross reactivity with putatively interfering organism occurring in the sample material (broiler feces) was tested. Also, several different E. *coli* isolates from chicken were tested on the presence of *ybbW* in order to ensure the sensitivity of the PCR. The results are listed in Table 11.

When testing other organisms than E. *coli* on the ybbW gene, unspecific amplifications occurred for almost all organisms. The specificity of the SYBR qPCR reactions was evaluated by melt peak and sequence analysis. All tested E. *coli* strains showed specific amplifications of *ybbW*.

**TABLE 11 INCLUSIVITY AND EXCLUSIVITY TESTS OF THE *YBBW* QPCR**

| Species | Strain | Origin | *YbbW* qPCR |
|---|---|---|---|
| **C. perfringens** | CP43 | Chicken | Unspecific amplification |
| **S. enterica subsp. enterica typhimurium** | DSM 17058 | Unknown, type strain | Unspecific amplification |
| | DSM 5569 | Unknown, control strain | Unspecific amplification |
| **C. jejuni** | DSM 24129 | Chicken | Negative |
| | DSM 24306 | Chicken | Negative |
| **Streptococcus gallinaceus** | DSM 15349 | Chicken sepsis | Unspecific amplification |
| **E. coli** | DSM 10779 | Diseased poultry | Specific amplification |
| | DSM 30083 | Urine | Specific amplification |
| | DSM 1103 | Clinical Isolate | Specific amplification |
| | BB007 | Chicken | Specific amplification |
| | BB008 | Chicken | Specific amplification |
| | BB009 | Chicken | Specific amplification |
| | BB010 | Chicken | Specific amplification |
| | BB011 | Chicken | Specific amplification |
| | BB012 | Chicken | Specific amplification |
| | BB013 | Chicken | Specific amplification |
| | BB014 | Chicken | Specific amplification |
| | BB015 | Chicken | Specific amplification |
| | BB016 | Chicken | Specific amplification |

This inclusivity and exclusivity of *ybbW* confirms the finding of Walker (2017) that *ybbW* is only found in E. *coli* but not in related species such as *Salmonella*.

CONCLUSIONS

The five selected APEC VFs are very abundant in fecal samples even in non-quantitative PCR reactions and are likely to be even more prevalent in a more sensitive qPCR approach.

Differences in concentration of the five VFs are even visible with the non-quantitative pentaplex PCR and allow a clear differentiation of a fecal sample from an APEC infected flock and a healthy flock.

For the fecal sample from the APEC infected flock it was confirmed that the five VFs originated from single pathogenic strain(s), carrying all five factors *ybbW* is a suitable reference gene for *E.* coli as it was exclusively found in E. *coli* strains and showed a similar concentration in different fecal samples from commercial broiler flocks in Germany and in the US.

Based on these results, the non-quantitative detection of an APEC VF set in a fecal sample does not enable the discrimination between infected and non-infected flocks, as the VFs occur with a high prevalence in broiler feces, at least with a low concentration. A quantitative method, which not only allows the numeric count of APEC factors, but also the relation of the absolute VF concentration to the overall *E. coli* (in forms of *ybbW*) load can give insight into the APEC risk of the flock or confirm an APEC outbreak when performance losses occur. Such an assay facilitates and accelerates the APEC diagnosis in broiler flocks and enables a whole flock analysis.

OCCURRENCE OF APEC VFS AND E. COLI REFERENCE GENE IN E.COLI ISOLATES FROM DIFFERENT COUNTRIES AND ORIGIN SPECIES

During evaluation of the APEC virulence factors and *E. coli* reference genes, 10 different wild type *E. coli* strains isolated from poultry organs or carcasses originating from different countries were analyzed regarding the presence of the five APEC markers and the quantity of the *ybbW* reference gene (

Table 13). All strains contained the reference gene with a similar concentration/Cq but different counts of the five VFs. All strains that showed 4/5 or 3/5 APEC VFs, were lacking the ompT gene or the ompT and the iroN gene. Also, all strains with exception for strain 5 contained hlyF and iss.

TABLE 13 OCCURRENCE OF APEC VFS (PENTAPLEX PCR) AND E. COLI REFERENCE GENE IN E. COLI ISOLATES FROM DIFFERENT COUNTRIES AND ORIGIN SPECIES

| Strain | | | APEC Analysis (Pentaplex+ybbW) | | | | | |
|---|---|---|---|---|---|---|---|---|
| No | Origin species | Origin country | iroN (553bp) | ompT (496bp) | hlyF (450bp) | iss (323bp) | iutA (302bp) | ybbW (Cq) |
| 1 | Chicken | Germany | - | - | + | + | + | 14.05 |
| 2 | Chicken | Poland | + | - | + | + | + | 15.33 |
| 3 | Poultry | Germany | - | - | - | - | - | 14.51 |
| 4 | Turkey | Germany | - | - | + | + | - | 14.30 |
| 5 | Turkey | Germany | - | - | - | - | + | 14.85 |
| 6 | Chicken | Poland | + | + | + | + | + | 15.46 |
| 7 | Turkey | Germany | - | - | + | + | - | 14.12 |
| 8 | Chicken | Germany | - | - | + | + | + | 14.23 |
| 9 | Turkey | Netherlands | - | - | + | + | + | 17.44 |
| 10 | Turkey | Germany | + | + | + | + | + | 16.91 |

Based on this subset of strains, it is enough to only analyze the targets ompT and iroN, because the call of iroN is associated with 4/5 positive VFs and the call of both ompT and the iroN with 5/5 positive VFs.

DETECTION OF APEC IN AIR DUST SAMPLES

Air dust samples from a commercial broiler flock in Germany suffering an APEC outbreak diagnosed with polyarthritis were collected by placing petri dishes at four different points in the stable and incubating them there for approximately 1 h. The dust gathered in the dishes was collected a sterile swap and applied to nucleic acid extraction by placing it in a 2 ml tube filled with 800 µl Lysis buffer from the ScreenFloX® PCR Nucleic acid extraction kit, 5 zirconia beads (2 mm diameter) and 1 glass bead (6 mm diameter). Each sample was incubated at 70° C for 20 min, horizontally mixed for 15 min at 20 Hz and centrifuged down for 5 min at 2000 g. 500 µl of the pretreated sample were applied for DNA extraction with the ScreenFloX® PCR Nucleic acid extraction kit according to the IFU and afterwards analyzed with the pentaplex PCR.

The resulting agarose gel is shown in Figure 6. Even though the air dust samples (lane 1-4) showed very faint bands of the PCR products compared to the positive fecal sample (lane 5), the three larger APEC virulence factors iroN, ompT and hlyF are visible and distinguishable from the NTC (lane 7) in at least 3 of the 4 dust samples (lane 2-4). Also, one or two products of the smaller sizes (around 300 bp) are perceivable.

The experiment shows that detecting APEC VFs in air dust samples is possible even though the applied collection method only led to a small amount of dust input. The sensitivity of the approach can be easily improved by applying devices developed for air sample collection, which result in significantly higher sample inputs.

**Claims**

1. Method for determining the risk of an Avian Pathogenic E. coli (APEC) infection in an avian flock, the method comprising

   determining the quantitative ratio of the amount of specific virulence factors (VF), each with respect to the amount of E. coli reference gene ybbW (SEQ ID NO: 6) or functional fragments thereof, in a pooled sample deriving from the avian flock,
   wherein the specific virulence factors include at least the genes iroN (SEQ ID NO.: 1) and ompT (SEQ ID NO: 2) or functional fragments thereof.

2. The method according to claim 1, wherein the specific virulence factors further include at least one gene selected from the group consisting of hlyF (SEQ ID NO: 3), iss (SEQ ID NO: 4), iutA (SEQ ID NO: 5), or functional fragments thereof.

3. The method according to any one of the preceding claims, wherein the pooled sample deriving from the avian flock is an environmental sample collected in the stable housing the avian flock.

4. The method according to claim 3, wherein the environmental sample is selected from excremental material, litter material, air dust material, or combinations thereof.

5. The method according to any one of claims 3 or 4, wherein the environmental sample is excremental material.

6. The method according to claim 5, wherein the excremental sample material is fecal material.

7. The method according to claim 6, wherein the fecal material is a composite sample of randomly collected individual fecal samples.

8. The method according to any one of the preceding claims, wherein the E. coli reference gene and the APEC virulence factors are detected and quantified via qPCR.

9. Environmental screening kit comprising primers and optionally comprising probes for detecting and quantifying at least iroN (SEQ ID NO.: 1), ompT (SEQ ID NO: 2), and ybbW (SEQ ID NO: 6).

10. Environmental screening kit according to claim 9, further comprising primers and optionally further comprising probes for detecting and quantifying at least one gene selected from the group consisting of hlyF (SEQ ID NO: 3), iss (SEQ ID NO: 4), iutA (SEQ ID NO: 5).

**Patentansprüche**

1. Verfahren zur Bestimmung des Risikos einer APEC(Avian Pathogenic E. coli)-Infektion in einer Vogelschar, wobei das Verfahren

   Bestimmen des quantitativen Verhältnisses der Menge spezifischer Virulenzfaktoren (VF), jeweils in Bezug auf die Menge an E. coli-Referenzgen ybbW (SEQ ID NO: 6) oder funktionsfähigen Fragmenten davon, in einer von der Vogelschar stammenden Sammelprobe umfasst,
wobei die spezifischen Virulenzfaktoren wenigstens die Gene iroN (SEQ ID NO: 1) und ompT (SEQ ID NO: 2) oder funktionsfähige Fragmente davon umfassen.

2. Verfahren nach Anspruch 1, wobei die spezifischen Virulenzfaktoren ferner wenigstens ein Gen ausgewählt aus der Gruppe bestehend aus hlyF (SEQ ID NO: 3), iss (SEQ ID NO: 4), iutA (SEQ ID NO: 5) oder funktionsfähige Fragmente davon umfassen.

3. Verfahren nach einem der vorhergehenden Ansprüche, wobei es sich bei der von der Vogelschar stammenden Sammelprobe um eine in der stabilen Behausung der Vogelschar gesammelte Umweltprobe handelt.

4. Verfahren nach Anspruch 3, wobei die Umweltprobe aus Exkrementmaterial, Einstreumaterial, Luftstaubmaterial oder Kombinationen davon ausgewählt ist.

5. Verfahren nach einem der Ansprüche 3 oder 4, wobei es sich bei der Umweltprobe um Exkrementmaterial handelt.

6. Verfahren nach Anspruch 5, wobei es sich bei dem Exkrementprobenmaterial um Kotmaterial handelt.

7. Verfahren nach Anspruch 6, wobei es sich bei dem Kotmaterial um eine Mischprobe zufällig gesammelter einzelner Kotproben handelt.

8. Verfahren nach einem der vorhergehenden Ansprüche, wobei das E. coli-Referenzgen und die APEC-Virulenzfaktoren über qPCR nachgewiesen und quantifiziert werden.

9. Umwelt-Screening-Kit, umfassend Primer und gegebenenfalls Sonden zum Nachweisen und Quantifizieren von wenigstens iroN (SEQ ID NO: 1), ompT (SEQ ID NO: 2) und ybbW (SEQ ID NO: 6).

10. Umwelt-Screening-Kit nach Anspruch 9, ferner umfassend Primer und gegebenenfalls ferner umfassend Sonden zum Nachweisen und Quantifizieren wenigstens eines aus der Gruppe bestehend aus hlyF (SEQ ID NO: 3), iss (SEQ ID NO: 4), iutA (SEQ ID NO: 5) ausgewählten Gens.

**Revendications**

1. Procédé pour la détermination du risque d'une infection par E. coli aviaire pathogène (APEC) dans un troupeau aviaire, le procédé comprenant

la détermination du rapport quantitatif de la quantité de facteurs de virulence spécifique (VF) chacun par rapport à la quantité d'un gène de référence de E. coli ybbW (SEQ ID NO: 6) ou de fragments fonctionnels correspondants, dans un échantillon groupé issu du troupeau aviaire,
les facteurs de virulence spécifique comprenant au moins les gènes iroN (SEQ ID NO: 1) et ompt (SEQ ID NO: 2) ou des fragments fonctionnels correspondants.

2. Procédé selon la revendication 1, les facteurs de virulence spécifique comprenant en outre au moins un gène choisi dans le groupe constitué par hlyF (SEQ ID NO: 3), iss (SEQ ID NO: 4), iutA (SEQ ID NO: 5), ou des fragments fonctionnels correspondants.

3. Procédé selon l'une quelconque des revendications précédentes, l'échantillon groupé issu du troupeau aviaire étant un échantillon environnemental collecté dans l'étable accueillant le troupeau aviaire.

4. Procédé selon la revendication 3, l'échantillon environnemental étant choisi parmi une matière d'excréments, une matière de litière, une matière de poussière dans l'air, ou des combinaisons correspondantes.

5. Procédé selon l'une quelconque des revendications 3 et 4, l'échantillon environnemental étant une matière d'excréments.

6. Procédé selon la revendication 5, la matière d'échantillon d'excréments étant une matière fécale.

**7.** Procédé selon la revendication 6, la matière fécale étant un échantillon composite d'échantillons fécaux individuels collectés de manière aléatoire.

**8.** Procédé selon l'une quelconque des revendications précédentes, le gène de référence de E. coli et les facteurs de virulence d'APEC étant détectés et quantifiés via une qPCR.

**9.** Kit de criblage environnemental comprenant des amorces et éventuellement comprenant des sondes pour la détection et la quantification d'au moins iroN (SEQ ID NO: 1), ompT (SEQ ID NO: 2) et ybbW (SEQ ID NO: 6).

**10.** Kit de criblage environnemental selon la revendication 9, comprenant en outre des amorces et éventuellement comprenant en outre des sondes pour la détection et la quantification d'au moins un gène choisi dans le groupe constitué par hlyF (SEQ ID NO: 3), iss (SEQ ID NO: 4), iutA (SEQ ID NO: 5).

FIG. 1:

FIG. 2:

M: marker/ladder

1-16: single *E. coli* isolates

+K: positive control (fecal DNA)

H20: negative control/non template control

FIG. 3:

FIG. 4:

FIG. 5:

FIG. 6:

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JOHNSON, T. J. et al.** Identification of Minimal Predictors of Avian Pathogenic Escherichia coli Virulence for Use as a Rapid Diagnostic Tool. *J Clin Microbiol,* 2008, vol. 46 (12), 3987-3996
- **SCHOULER, C. et al.** Diagnostic strategy for identifying avian pathogenic Escherichia coli based on four patterns of virulence genes. *J Clin Microbiol,* 2012, vol. 50 (5), 1673-1678
- **WALKER, D. I. et al.** A highly specific Escherichia coli qPCR and its comparison with existing methods for environmental waters. *Water Research,* 2017, vol. 126, 101-110